# EUROPÄISCHE PATENTSCHRIFT

(11) **EP 4 028 379 B1**
(45) Veröffentlichungstag und Bekanntmachung des Hinweises auf die Patenterteilung: **19.11.2025**
(21) Anmeldenummer: 20767558.8
(22) Anmeldetag: 09.09.2020
(51) Int. Cl.: C07C 29/44, C07C 33/50

(54) **VERBESSERTES VERFAHREN ZUR HERSTELLUNG VON CYCLOPROPYLVERBINDUNGEN AUS ALKENEN**
IMPROVED METHOD FOR THE PREPARATION OF CYCLOPROYL COMPOUNDS FROM ALKENES
PROCÉDÉ AMÉLIORÉ DE FABRICATION DE COMPOSÉS CYCLOPROPYLE À PARTIR D'ALCÈNES

(30) Priorität: 12.09.2019 EP 19196901
(43) Veröffentlichungstag der Anmeldung: 20.07.2022
(73) Patentinhaber: Saltigo GmbH, 51369 Leverkusen (DE)
(72) Erfinder: GROSSMANN, Andre, 50735 Köln (DE); SCHLUMMER, Björn, 53225 Bonn (DE)
(74) Vertreter: Matzke, Michael
(86) Internationale Anmeldenummer: PCT/EP2020/075207
(87) Internationale Veröffentlichungsnummer: WO 2021/048210

(56) Entgegenhaltungen:
- EP-A2- 0 321 409
- WO-A1-2017/024126
- CN-A- 109 553 583
- CHARETTE A B ET AL: "Simmons-Smith cyclopropanation reaction", 20 January 2001, ORGANIC REACTIONS, JOHN WILEY & SONS, INC, PAGE(S) 1 - 415, ISBN: 978-0-471-10590-9, XP002788506

## Beschreibung

Die Erfindung betrifft ein verbessertes Verfahren zur Herstellung von 1-(4-Chlorphenyl)-2-cyclopropyl-propan-1-ol aus 4-(4-Chlorphenyl)-3-methyl-but-1-en-4-ol durch Umsetzung des Alkens in Gegenwart von Bromchlormethan, elementarem Zink und elementarem Kupfer oder Kupferverbindungen (Cyclopropanierungsreaktion) in Abwesenheit von Organosiliciumverbindungen und/oder ohne Zugabe von Organosiliciumverbindungen.

Die Umsetzung von Alkenen zu den homologen Cyclopropylverbindungen ist unter dem Namen Simmons-Smith-Reaktion bekannt, in der üblicherweise Dihalogenalkane, meistens Diiodmethan, in Gegenwart von Zink und Kupfer umgesetzt werden. Dabei wird aus dem mit Kupfer aktiviertem Zink (Zink-Kupfer-Paar) intermediär ein Zinkcarben (Carbenoid) erzeugt, dessen Methylengruppe an die Doppelbindung des Alkens addiert wird.

Während Cyclopropanierungsreaktionen in Gegenwart von Diiodmethan aufgrund dessen hoher Reaktivität, die für die Bildung der Zinkcarben-Zwischenstufe wohl erforderlich ist, häufig hohe Ausbeuten liefern, verlaufen Reaktionen von chlor- oder bromhaltigen Alkanen als Carbenvorstufe in der Regel deutlich ungünstiger. Deshalb sind im Stand der Technik Varianten dieser Reaktion, beispielsweise für Dibrommethan als Dihalogenalkan beschrieben, bei denen das Zink bzw. das Zink-Kupfer-Paar zusätzlich durch Ultraschall (J. Org. Chem. 50, 1985, 4640; Friedrich et. al.), durch Titantetrachlorid-Katalyse (J. Org. Chem. 1989, 54, 2388; Friedrich et al.). Eine Herstellung der Verbindung der Formel (II) ist in EP 0321409 A2 offenbart. Dort erfolgt die Cyclopropanierung des Alkens der Formel (I) mit einem Zink-Kupfer-Paar und Dibrommethan in Gegenwart des metallorganischen Katalysators Natriumdihydrido-bis(2-ethoxymethoxy)aluminat [sodium dihydrido-bis(2-ethoxymethoxy)aluminate; SDBA].

Für Reaktionen mit Bromchlormethan als Dihalogenalkan gibt es ebenfalls Varianten, die nur mit zusätzlicher Aktivierung des Zinks bzw. das Zink-Kupfer-Paares die gewünschten Cyclopropylverbindungen in akzeptablen Ausbeuten liefern.

In J. Org. Chem., 56 (10), 3255, 1991 (Sibille et al.) wird beispielsweise eine elektrochemische Cyclopropanierung von Allylalkoholen mit Dibrommethan oder Bromchlormethan unter Verwendung einer Zinkanode und einer Kohlenstofffaserkathode beschrieben. Diese Reaktion hat jedoch den Nachteil, dass die Durchführung in größerem technischen Maßstab teuer, aufwändig und schwierig zu kontrollieren ist.

Als Stand der Technik ist weiterhin in der WO 2017/024126 ein Verfahren beschrieben, in dem als Dihalogenalkane anstatt Diiodmethan Dibrommethan oder Bromchlormethan verwendet werden und in dem das Zink bzw. das Zink-Kupfer-Paar zusätzlich aktiviert wird. Als Aktivatoren sind hier Haloalkylsilane, beispielsweise Chlortrimethylsilan, zwingend erforderlich, um akzeptable Ausbeuten zu erhalten. Auch dieses Verfahren ist in größerem Maßstab aufgrund der erforderlichen siliziumhaltigen Aktivatoren aufwändiger und teurer in der Durchführung als die klassische Simmons-Smith-Reaktion, die aber aufgrund des hohen Preises des erforderlichen Diiodmethans ebenfalls im größerem Maßstab nicht wirtschaftlich ist.

Die nach dem erfindungsgemäßen Verfahren hergestellten Cyclopropylverbindungen sind beispielsweise Zwischen- oder Endstufen für unterschiedliche gewerbliche Anwendungen wie Aromastoffe oder Agrochemikalien. Die Verbindung der Formel (II) ist beispielsweise ein Zwischenprodukt für das Azolfungizid Cyproconazol (DE 3406993, CN 105820128, CN 101857576).

Es besteht also nach wie vor das Bedürfnis und somit die technische Aufgabe, eine Variante der Simmons-Smith-Reaktion bereitzustellen, die die Nachteile des Standes der Technik nicht aufweist.

Überraschenderweise wurde nun ein Verfahren zur Herstellung der Cyclopropylverbindung der Formel (II) 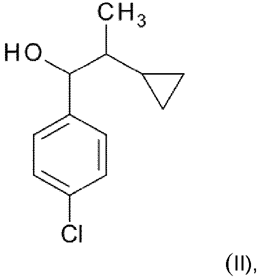 gefunden, umfassend die Umsetzung des Alkens der Formel (I), 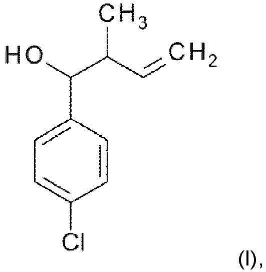 mit Bromchlormethan in Gegenwart von (i) elementarem Zink, (ii) katalytisch wirksamen Mengen von elementarem Kupfer und/oder Kupfer(I)verbindungen und/oder Kupfer(II)verbindungen, (iii) und zumindest einem Lösungsmittel, dadurch gekennzeichnet, dass die Umsetzung des Alkens in Abwesenheit von Organosiliciumverbindungen, bevorzugt in Abwesenheit von Haloalkylsilanen, und/oder ohne Zugabe von Organosiliciumverbindungen, bevorzugt ohne Zugabe von Haloalkylsilanen, erfolgt, und das elementare Zink einen Gehalt von höchstens 0,005 Gew.% (50 ppm) Blei, bevorzugt von höchstens 0,002 Gew.% (20 ppm) Blei, aufweist.

Bei der Cyclopropanierung entsteht aus dem Alken die entsprechende homologe Cyclopropylverbindungen, die formal als Additionsprodukte eines Carbens "CH2" an die Doppelbindung des Alkens zu verstehen sind.

Das als Halogenalkan eingesetzte Bromchlormethan (CH₂BrCl) ist kommerziell erhältlich oder kann mit geeigneten Verfahren selbst hergestellt werden und weist bevorzugt eine Reinheit von mindestens 98 Gew.% und einem Wassergehalt von kleiner 0,02 Gew.%, bevorzugt von kleiner 0,007 Gew.%, auf.

Das als Halogenalkan eingesetzte Bromchlormethan kann beispielsweise auch als Mischung mit Dibrommethan eingesetzt werden. Jedoch wird die Reaktion mit zunehmendem Anteil an Dibrommethan unwirtschaftlicher. Zusätzlich sinken die erzielten Ausbeuten mit zunehmenden Anteil an Dibrommethan.

Das erfindungsgemäße Verfahren wird in Gegenwart von elementarem Zink durchgeführt. Das elementare Zink liegt dabei üblicherweise in feinteiliger Form vor. Unter feinteiliger Form wird im Sinne der Erfindung ein pulverförmiger, körniger oder granularer Feststoff verstanden, der bevorzugt leicht schüttbar und damit gut dosierbar ist. Für das erfindungsgemäße Verfahren ist eine Voraussetzung, dass das elementare Zink einen Gehalt an Blei von höchstens 0,005 Gew.%, bevorzugt von höchstens 0,002 Gew.% Blei, aufweist. Der Bleigehalt in diesem Konzentrationsbereich wird dabei üblicherweise mit Atomadsorptionsspektrometrie gemessen.

Bevorzugt wird in das erfindungsgemäße Verfahren elementares Zink in feinteiliger Form, d.h. als elementares Zink mit einer Partikelgrößenverteilung D90_{Masse} von nicht mehr als 0,5 mm eingesetzt. Die Partikelgrößenverteilung D90_{Masse} von nicht mehr als 0,5 mm bedeutet im Sinne der Erfindung, dass 90 Gew.% der zu messenden Probe eine Partikelgröße von 0,5 mm und kleiner aufweisen. Die Partikelgrößenverteilung D90_{Masse} wird üblicherweise durch Siebung und nachfolgende Wägung der gesiebten Partikel bestimmt und weist eine hohe Genauigkeit auf. In einer weiteren Ausführungsform weist das metallische Zink bevorzugt eine Siebverteilung von höchstens 15 Gew.% einer Teilchengröße größer als 250 µm, von 25 bis 50 Gew.% einer Teilchengröße von 150 bis 250 µm, von 30 bis 60 Gew.% einer Teilchengröße von 45 bis 150 µm und höchstens 15 Gew.% einer Teilchengröße kleiner als 45 µm auf, wobei jedoch insgesamt eine Partikelgrößenverteilung D90_{Masse} von nicht mehr als 0,5 mm eingehalten wird.

Weiterhin bevorzugt weist das elementare Zink neben einem Bleigehalt von höchstens 0,005 Gew.% Blei, bevorzugt von höchstens 0,002 Gew.% Blei, einen Gesamtgehalt an anderen Metallen von höchstens 1 Gew.%, bevorzugt von 0,1 Gew.%, besonders bevorzugt von 0,05 Gew.% auf. Diese anderen Metalle sind beispielsweise Cadmium, Eisen, Quecksilber, Wismut oder Indium. Die Bestimmung der Gehalte der anderen Metalle erfolgt in der Regel durch Atomadsorptionsspektrometrie.

Weiterhin bevorzugt weist das elementare Zink einen Zinkgehalt von mindestens 99,0 Gew.%, bevorzugt von mindestens 99,9 Gew.% auf. Die Bestimmung des Zink-Gehalts erfolgt in der Regel durch Atomadsorptionsspektrometrie.

Dabei werden in das erfindungsgemäße Verfahren bevorzugt von 1,5 bis 4 mol, bevorzugt von 2 bis 3 mol, metallisches Zink bezogen auf 1,0 mol der Verbindung der Formel (I), eingesetzt.

In das erfindungsgemäße Verfahren werden entweder elementares Kupfer, Kupfer(I)verbindungen oder Kupfer(II)verbindungen, oder Mischungen davon, eingesetzt.

Dabei weist das elementare Kupfer bevorzugt neben einem Bleigehalt von höchstens 0,005 Gew.% Blei, bevorzugt von höchstens 0,002 Gew.% Blei, einen Gesamtgehalt an anderen Metallen von höchstens 1 Gew.%, bevorzugt von 0,1 Gew%, besonders bevorzugt von 0,05 Gew.% auf. Diese anderen Metalle sind beispielsweise Cadmium, Eisen, Quecksilber, Wismut oder Indium. Die Bestimmung der Gehalte der anderen Metalle erfolgt in der Regel durch Atomadsorptionsspektrometrie.

Wenn in das erfindungsgemäße Verfahren Kupfer(I)verbindungen eingesetzt werden, ist dies bevorzugt Kupfer(I)chlorid. Werden in das erfindungsgemäße Verfahren Kupfer(II)verbindungen eingesetzt, sind dies bevorzugt Kupfer(II)chlorid, Kupfer(II)phosphat, Kupfer(II)carbonat oder Mischungen davon.

Die Menge an eingesetztem Kuper oder Kupferverbindung hängt von der Anzahl der Kohlenstoff-Kohlenstoff-Doppelbindungen des Alkens ab, die cyclopropaniert werden sollen. Wenn das Alken nur eine Doppelbindung aufweist, die cyclopropaniert werden soll, wird die katalytisch wirksame Menge an elementarem Kupfer und/oder Kupfer(I)verbindungen und/oder Kupfer(II)verbindungen, in einer Gesamtmenge von 0,001 bis 0,1 mol, bevorzugt von 0,001 bis 0,01 mol, bezogen auf 1,0 mol der Verbindung der Formel (I), eingesetzt. Pro weiterem Mol aliphatischer Kohlenstoff-Kohlenstoff-Doppelbindung in dem Alken, die cyclopropaniert werden soll, wird das entsprechende ganzzahlige Vielfache der oben genannten Menge eingesetzt.

Ebenfalls hängt die Menge des in das erfindungsgemäße Verfahren eingesetzte Bromchlormethan von der Anzahl der zu cyclopropanierenden Doppelbindungen in dem Alken ab. Bei einer Doppelbindung, wie es in Verbindung der Formel (I) der Fall ist, werden beispielsweise von 1 bis 3 mol, bevorzugt von 1,7 bis 2,2 mol, Bromchlormethan, bezogen auf 1,0 mol der Verbindung der Formel (I), eingesetzt.

Das erfindungsgemäße Verfahren wird in Abwesenheit von Organosiliciumverbindungen durchgeführt. "In Abwesenheit von Organosiliciumverbindungen", "bevorzugt in Abwesenheit von Haloalkylsilanen", bedeutet, dass während der gesamten Umsetzung des Alkens ein Anteil von höchstens 0,1 Gew.%, vorzugsweise von höchstens 0,02 Gew.%, Organosiliciumverbindungen, bevorzugt von höchstens 0,1 Gew.%, vorzugsweise von 0,02 Gew.%, Haloalkylsilanen, bezogen auf die Masse des elementaren Zinks, in der Reaktionsmischung vorliegt. In einer alternativen Ausführungsform erfolgt die Durchführung des erfindungsgemäßen Verfahrens ohne Zugabe von Organosiliciumverbindungen, bevorzugt ohne Zugabe von Haloalkylsilanen. "Ohne Zugabe von Organosiliciumverbindungen" bedeutet im Sinne der Erfindung, dass weder der Verbindung der Formel (I), noch dem Bromchlormethan, noch der Verbindung der Formel (II), noch dem elementaren Zink, noch dem elementaren Kupfer und/oder den Kupfer(I)verbindungen und/oder den Kupfer(II)verbindungen, noch dem Lösungsmittel, noch weiteren dem Reaktionsgemisch zugegebenen Einsatzstoffen und noch der Reaktionsmischung selbst zu irgend einem Zeitpunkt der Umsetzung oder vorher Organosiliciumverbindungen, bevorzugt Haloalkylsilane, zugegeben wurden. Ein Anteil von mehr als 0,1 Gew.%, bevorzugt von mehr als 0,02 Gew.%, Organosiliciumverbindungen, bezogen auf die Masse des elementaren Zinks, ist in der Reaktionsmischung nur denkbar, wenn eine entsprechende Menge an Organosiliciumverbindungen, bevorzugt an Haloalkylsilanen, der Reaktionsmischung oder einem der Einsatzstoffe vor oder während der Reaktion zugegeben wird. Als natürliche Verunreinigung der Einsatzstoffe Alken, Bromchlormethan, elementares Zink, elementares Kupfer und/oder Kupfer(I)verbindungen und/oder Kupfer(II)verbindungen, und (iii) Lösungsmittel treten Organosiliciumverbindungen nicht auf. Bevorzugt sind Organosiliciumverbindungen Haloalkylsilane. Haloalkylsilane sind beispielsweise Chlortrialkylsilane. Bevorzugte Vertreter der Chlortrialkylsilane sind Chlortrimethylsilan, Chlortriethylsilan, Chlortributylsilan, Chlortriisobutylsilan oder Chlortrihexylsilan.

Als Lösungsmittel können in dem erfindungsgemäßen Verfahren beispielsweise Ether und/oder aromatische Kohlenwasserstoffe eingesetzt werden. Bevorzugte Ether sind Diethylether, 1,2-Dimethoxyethan, Methyl-tert-butylether, Tetrahydrofuran, Cyclopentylmethylether und Mischungen davon. Ein bevorzugter aromatischer Kohlenwasserstoff ist Toluol. Vorzugsweise werden von 0,7 bis 1,5 mol, bevorzugt von 1,0 bis 1,2 mol, Ether und/oder 2,0 bis 6,0 mol, bevorzugt von 2,0 bis 3,0 mol, aromatischer Kohlenwasserstoff pro mol Alken eingesetzt.

Im Folgenden wird das erfindungsgemäße Verfahren detaillierter beschrieben:
Alle Schritte dieser Reaktion vor der Hydrolyse des Reaktionsgemisches werden üblicherweise unter Schutzgasatmosphäre durchgeführt. Geeignete Schutzgase sind beispielsweise Stickstoff oder Argon.

In einer Ausführungsform des erfindungsgemäßen Verfahrens werden zunächst die Einsatzstoffe Verbindung der Formel (I), elementares Zink, elementares Kupfer und/oder Kupfer(I)verbindungen und/oder Kupfer(II)verbindungen, sowie Lösungsmittel bei Temperaturen von 15 bis 80 °C, bevorzugt von 50 bis 70 °C von in ein Reaktionsbehältnis gegeben. Dabei entsteht eine heterogene Zweiphasen-Mischung, die durch mechanische oder hydraulische Durchmischung zunächst in eine möglichst homogene Zweiphasen-Mischung umgewandelt wird. Da die Vermischung der oben genannten Einsatzstoffe bei Umgebungstemperatur nicht exotherm ist, kann die Zugabe der einzelnen Einsatzstoffe diskontinuierlich oder kontinuierlich und in beliebiger Reihenfolge erfolgen. Bevorzugt wird das Lösungsmittel vorgelegt und die weiteren Einsatzstoffe nachfolgend unter Vermischung dazugegeben. Dadurch kann von Verklumpen der festen Einsatzstoffe vermieden werden. Nach oder während der Zugabe der Einsatzstoffe kann die Temperatur auf 85°C erhöht werden. Anschließend wird Bromchlormethan zu der Reaktionsmischung gegeben. Dies erfolgt beispielsweise diskontinuierlich oder kontinuierlich, bevorzugt kontinuierlich. Bevorzugt werden zunächst 0,05 bis 0,1 mol Bromchlormethan pro mol Alken, bevorzugt Verbindung der Formel (I), der Reaktionsmischung zugegeben. Innerhalb von 1 bis 240 Minuten beginnt dann die Reaktion durch eine exotherme Wärmeentwicklung, wodurch die Temperatur der Reaktionsmischung um 1,5 bis 10 °C ansteigen kann. Nach Beginn der exothermen Wärmeentwicklung wird durch Kühlung, bevorzugt durch externe Kühlung des Reaktionsbehältnisses, die Temperatur der Reaktionsmischung in einem Bereich von 55 bis 85 °C, bevorzugt in einem Bereich von 60 bis 80 °C, besonders bevorzugt in einem Bereich von 67 bis 73°C, gehalten. Dabei wird die Zugabe des Bromchlormethans fortgesetzt, bis die Gesamtmenge an Bromchlormethan zugegeben wurde. Während der Zugabe des Bromchlormethans können, falls die Exothermie und damit der Reaktionsfortschritt geringer wird, elementares Kupfer und/oder Kupfer(I)verbindungen und/oder Kupfer(II)verbindungen, dem Reaktionsgemisch zudosiert werden. Üblicherweise werden dadurch die Exothermie und damit der Reaktionsfortschritt wieder gesteigert. Nach beendeter Zugabe der Gesamtmenge an Bromchlormethan wird das Reaktionsgemisch während und nach der Zugabe von Bromchlormethan noch 2 bis 5 Stunden bei einem Temperaturbereich von 55 bis 85 °C, bevorzugt in einem Bereich von 60 bis 80 °C, besonders bevorzugt von 65 bis 75°C, weiter vermischt. Dabei können Proben der Reaktionsmischung entnommen und nach einer Aufarbeitung auf den Gehalt an Alken der Formel (I), und/oder der Verbindung der Formel (II), gemessen werden. Wenn die Reaktion beendet ist, wird die Reaktionsmischung, d.h. die Mischung aller bis dahin zugegebenen Einsatzstoffe, als Rohmischung bezeichnet.

In einer bevorzugten Ausführungsform des erfindungsgemäßen Verfahrens werden 0,01 bis 5 Gew.%, bevorzugt von 0,1 bis 1 Gew.%, bezogen auf das Gesamtgewicht der Rohmischung, einer Rohmischung aus einer vorherigen Umsetzung, bezogen auf die eingesetzte Menge an Alken der Formel (I), dem Reaktionsgemisch zugegeben. Üblicherweise reicht dafür aus, die Reste der Rohmischung aus der vorherigen Umsetzung im Reaktor zu belassen. Die Rohmischung aus einer vorherigen Umsetzung entspricht der Reaktionsmischung nach beendeter Reaktion, aber vor der Hydrolyse der Mischung. Die Rohmischung aus einer vorherigen Umsetzung kann entweder zu Beginn der Zugabe der Einsatzstoffe Verbindung der Formel (I), elementares Zink, elementares Kupfer und/oder Kupfer(I)verbindungen und/oder Kupfer(II)verbindungen, sowie Lösungsmittel in dem Reaktionsbehältnis zugegeben werden, oder ist aus einer vorherigen Umsetzung in demselben Reaktionsbehältnis noch vorhanden. Die Rohmischung aus einer vorherigen Umsetzung kann aber auch zu einem späteren Zeitpunkt der Mischung der Einsatzstoffe Verbindung der Formel (I), elementarem Zink, elementarem Kupfer und/oder Kupfer(I)verbindungen und/oder Kupfer(II)verbindungen, sowie Lösungsmittel zugegeben werden. Dadurch wird der Beginn der exothermen Reaktion begünstigt. In einer weiteren Ausführungsform kann der Beginn der exothermen Reaktion auch begünstigt werden, indem das erfindungsgemäße Verfahren Umsetzung in Gegenwart von 0,1 bis 5 Gew.% Zinkhalogenid, bevorzugt Zinkchlorid oder Zinkbromid, bezogen auf die eingesetzte Menge an der Verbindung der Formel (I), durchgeführt wird. Dabei wird das Zinkhalogenid bevorzugt zu Beginn der Reaktion zugegeben.

Das Fortschreiten der Reaktion kann durch die Analyse von Proben festgestellt werden, die wie das Reaktionsgemisch aufgearbeitet worden sind. Der Gehalt an Edukt und Produkt kann dabei üblicherweise mittels HPLC oder Gaschromatografie, entweder als Flächenprozent ohne externen Standard oder als Gewichtsprozent mit externem Standard, ermittelt werden.

Nach beendeter Reaktion wird die Rohmischung üblicherweise hydrolysiert. Dazu werden beispielsweise 1 bis 5 kg Wasser und/oder Eis pro kg eingesetzter Verbindung der Formel (I) in einem separaten Reaktionsbehältnis vorgelegt und die Rohmischung, vorzugsweise unter mechanischer und/oder hydraulischer Vermischung, dazugegeben. Anschließend werden beispielsweise 0,5 bis 1,5 mol Chlorwasserstoff und/oder Bromwasserstoff, bevorzugt in Form von 20 bis 35 %iger wässriger Säure, pro mol eingesetzter Verbindung der Formel (I), zu dieser Mischung gegeben. Die dabei eintretende Hydrolyse verläuft exotherm. Bevorzugt wird bei der Hydrolyse darauf geachtet, dass die Temperatur des Gemisches nicht auf einen Bereich von über 35 °C steigt. Nach beendeter Zugabe des Chlorwasserstoffs beträgt der pH-Wert der Reaktionsmischung beispielsweise von 6,5 bis 7,5. Falls Wasser ganz oder teilweise durch Eis ersetzt wird, wird die Eismenge idealerweise so gewählt, dass nach beendeter Hydrolyse kein Eis mehr in der Reaktionsmischung vorhanden ist.

Nach beendeter Hydrolyse erfolgt beispielsweise eine Wäsche derart, dass zu der hydrolysierten Reaktionsmischung ein nicht mit Wasser mischbares Lösungsmittel, vorzugsweise ein aromatischer Kohlenwasserstoff, weiterhin bevorzugt Toluol, vorzugsweise unter mechanischer und/oder hydraulischer Vermischung, zugegeben wird. Nach Phasentrennung in eine organische Phase, die das cyclopropanierte Produkt enthält, und eine wässrige Phase kann eine Extraktion der wässrige Phase wiederholt werden. Dann werden die isolierten organischen Phasen bevorzugt vereint.

Die Verbindung der Formel (II) enthaltende organische Phase kann entweder als solche in eine neue Reaktion eingesetzt oder weiter aufgearbeitet werden, um die Verbindung der Formel (II) daraus zu isolieren.

Diese Isolierung erfolgt beispielsweise durch Abdestillieren von Lösungsmittel, sodass die Verbindung der Formel (II) als Sumpf verbleibt.

Überraschenderweise wird mit dem erfindungsgemäßen Verfahren auch in Abwesenheit von Organosiliciumverbindungen, bevorzugt in Abwesenheit von Haloalkylsilanen, und/oder ohne Zugabe von Organosiliciumverbindungen, bevorzugt ohne Zugabe von Haloalkylsilanen, die Verbindung der Formel (II) so in Ausbeuten von 85 bis 95% der Theorie erhalten.

### Beispiele:

### Beispiel 1 (erfindungsgemäß) [1-(4-Chlorphenyl)-2-cyclopropyl-propan-1-ol; Verbindung der Formel (II)]

Alle Schritte dieser Reaktion vor der Hydrolyse des Reaktionsgemisches wurden unter Stickstoffatmosphäre durchgeführt. In einem Reaktor 79,4 g (0,85 mol) Dimethoxyethan, 152,0 g 4-(4-Chlorphenyl)-3-methyl-but-1-en-4-ol [Verbindung der Formel (I)] (Gehalt 97,0 Gew.%, 0,75 mol), 193,3 g (2,07 mol) Toluol, 120 g Zinkpulver (1,84 mol) und 0,188 g (1,9 mmol) Kupfer(I)chlorid bei Umgebungstemperatur sowie 1-2 g nicht hydrolysierte Reaktionsmischung aus einer vorherigen Reaktion vorgelegt. Unter Rühren wurde das Gemisch auf 85 °C aufgeheizt. Bei Erreichen dieser Temperatur wurden 7,25 g Bromchlormethan innerhalb von 10 Minuten zudosiert. Nach 6 Minuten setzte eine exotherme Reaktion ein und die Reaktionsmischung erwärmte sich um 2 °C. Dann wurde durch Kühlung ein Temperaturbereich des Reaktionsgemisches von 67 bis 73 °C eingestellt. Nachfolgend wurden 179,1 g (1,38 mol) Bromchlormethan zu der Reaktionsmischung so zudosiert, dass die Temperatur des Reaktionsgemisches weiterhin im Bereich von 67 bis 73 °C verblieb. Dabei entwich aus der Reaktionsmischung gasförmiges Methylchlorid, das über einen Stickstoffstrom aus dem Reaktor in einen Wäscher ausgetragen wurde. Nach Beendigung der Zudosierung wurde die Reaktionsmischung 3 Stunden durch Rühren vermischt. Anschließend wurde die Temperatur der Reaktionsmischung unter Rühren auf 45 °C verringert. Zur Hydrolyse des Reaktionsgemisches wurde das so abgekühlte Reaktionsgemisch unter Rühren auf eine Mischung von 110 g Salzsäure (30 Gew.%) und 500 g Wasser gegeben, sodass der pH Wert der wässrigen Phase des zweiphasigen Gemisches von 6,5 bis 7,5 betrug. Nach Phasentrennung wurde die erste obere organische Phase abgetrennt. Die erste untere wässrige Phase wurde mit 50 g Toluol versetzt, verrührt und zur Phasentrennung ruhen gelassen. Die zweite obere organische Phase wurde abgetrennt und mit der ersten oberen organischen Phase vereinigt. Das Lösungsmittel wurde aus der vereinigten organischen Phase destillativ bei 90 °C und 20 hPa abgetrennt und das Produkt (1-(4-Chlorphenyl)-2-cyclopropyl-propan-1-ol) (168,4 g, Gehalt: 85,4 Gew.%) als Rohprodukt in einer Ausbeute von 91 % der Theorie erhalten.

**Tabelle 1: Die Versuche gemäß der Beispiele 2 und 3 wurden analog der Vorschrift des Beispiels 1, jedoch mit den in der Tabelle angegebenen Parametern, durchgeführt.**

| **Beispiel** | **Reaktionstemperatur** | **Moläquivalente Zink ^{*)}** | **Katalysator** | **Molprozent Katalysa tor ^{*)}** | **Methylenkomponente / Moläquivalente ^{*)}** | **Ausbeute (% der Theorie)** |
|---|---|---|---|---|---|---|
| 1 | 67 bis 73 °C | 2,45 | CuCl | 0,25 | Bromchlormethan / 1,91 | 91 |
| 2 | 85 °C | 2,45 | CuCl | 0,25 | Dibrommethan / 1,91 | 82 |
| 3 | 85 °C | 2,45 | CuCl | 0,25 | Dichlormethan / 1,91 | 0 |

| | | | | | | |
|---|---|---|---|---|---|---|
| *) bezogen auf Alken | | | | | | |

## Patentansprüche

1. Verfahren zur Herstellung von Cyclopropylverbindungen der Formel (II), 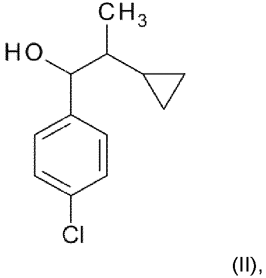 umfassend die Umsetzung eines Alkens der Formel (I), 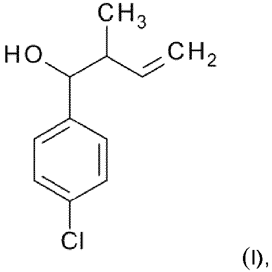 mit Bromchlormethan in Gegenwart von (i) elementarem Zink, (ii) katalytisch wirksamen Mengen von elementarem Kupfer und/oder Kupfer(I)verbindungen und/oder Kupfer(II)verbindungen, und (iii) zumindest einem Lösungsmittel, **dadurch gekennzeichnet, dass** die Umsetzung des Alkens ohne Zugabe von Organosiliciumverbindungen, bevorzugt ohne Zugabe von Haloalkylsilanen, erfolgt, und das elementare Zink einen Gehalt von höchstens 0,005 Gew.% Blei, bevorzugt von höchstens 0,002 Gew.% Blei, aufweist.

2. Verfahren nach Anspruch 1, wobei das elementare Zink eine Partikelgrößenverteilung D90_{Masse} von nicht mehr als 0,5 mm aufweist.

3. Verfahren nach einem der Ansprüche 1 oder 2, wobei das elementare Zink einen Gesamtgehalt an anderen Metallen von höchstens 1 Gew.% aufweist

4. Verfahren nach einem der Ansprüche 1 bis 3, wobei das elementare Kupfer einen Gesamtgehalt an anderen Metallen von höchstens 1 Gew.% aufweist.

5. Verfahren nach einem der Ansprüche 1 bis 4, wobei die Kupfer(I)verbindungen ausgewählt aus Kupfer(I)chlorid, Kupfer(II)chlorid, Kupfer(II)phosphat, Kupfer(II)carbonat oder Mischungen davon sind.

6. Verfahren nach einem der Ansprüche 1 bis 5, wobei das Haloalkylsilan ein Chlortrialkylsilan ist.

7. Verfahren nach einem der Ansprüche 1 bis 6, wobei das Chlortrialkylsilan ausgewählt aus Chlortrimethylsilan, Chlortriethylsilan, Chlorotributylsilan, Chlorotriisobutylsilan, Chlorotrihexylsilan und Mischungen davon ist.

8. Verfahren nach einem der Ansprüche 1 bis 7, wobei das zumindest eine Lösungsmittel ein Ether, bevorzugt ausgewählt aus Diethylether, 1,2-Dimethoxyethan, Methyl-tert-butylether, Tetrahydrofuran, Cyclopentylmethylether und Mischungen davon, und/oder ein aromatischer Kohlenwasserstoff, bevorzugt Toluol, ist.

9. Verfahren nach einem der Ansprüche 1 bis 8, wobei das Bromchlormethan eine Reinheit von mindestens 98 Gew.% und/oder einen Wassergehalt von kleiner 0,02 Gew.% aufweist.

10. Verfahren nach einem der Ansprüche 1 bis 9, wobei von 1,5 bis 4 mol, bevorzugt von 2 bis 3 mol, metallisches Zink bezogen auf 1,0 mol des Alkens, bevorzugt bezogen auf 1,0 mol der Verbindung der Formel (I), eingesetzt werden.

11. Verfahren nach einem der Ansprüche 1 bis 10, wobei die katalytisch wirksame Menge an elementarem Kupfer und/oder Kupfer(I)verbindungen und/oder Kupfer(II)verbindungen, in einer Gesamtmenge von 0,001 bis 0,1 mol, bevorzugt von 0,001 bis 0,01 mol, bezogen auf 1,0 mol des Alkens, bevorzugt bezogen auf 1,0 mol der Verbindung der Formel (I), eingesetzt werden.

12. Verfahren nach einem der Ansprüche 1 bis 11, wobei, von 1 bis 3 mol, bevorzugt von 1,7 bis 2,2 mol, Bromchlormethan bezogen auf 1,0 mol des Alkens, bevorzugt bezogen auf 1,0 mol der Verbindung der Formel (I), eingesetzt werden.

13. Verfahren nach einem der Ansprüche 1 bis 12, wobei die Reaktionstemperatur von 55 bis 85 °C, bevorzugt von 60 bis 80 °C, besonders bevorzugt während und nach der Zugabe von Bromchlormethan von 65 bis75 °C, beträgt.

14. Verfahren nach einem der Ansprüche 1 bis 13, wobei das Alken, vorzugsweise Verbindung der Formel (I), Bromchlormethan, elementarem Zink, katalytisch wirksamen Mengen von elementarem Kupfer und/oder Kupfer(I)verbindungen und/oder Kupfer(II)verbindungen, und zumindest ein Lösungsmittel, zur Herstellung der Cyclopropylverbindung, bevorzugt der Verbindung der Formel (II), vermischt werden, wobei eine Rohmischung entsteht, und die Rohmischung nach beendeter Herstellung hydrolysiert wird.

15. Verfahren nach einem der Ansprüche 1 bis 14, wobei die Herstellung der Cyclopropylverbindung, bevorzugt der Verbindung der Formel (II), in Gegenwart von 0,1 bis 5 Gew.%, bezogen auf das Gesamtgewicht der Rohmischung, einer Rohmischung aus einer vorherigen Herstellung der Cyclopropylverbindung, bevorzugt der Verbindung der Formel (II), bezogen auf die eingesetzte Menge des Alkens, bevorzugt bezogen auf die eingesetzte Menge der Verbindung der Formel (I), erfolgt.

## Claims

1. Process for preparing cyclopropyl compounds of formula (II), 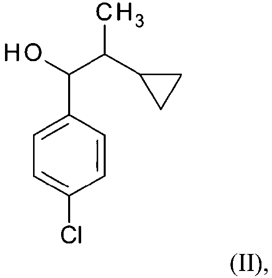 comprising the reaction of an alkene of formula (I), 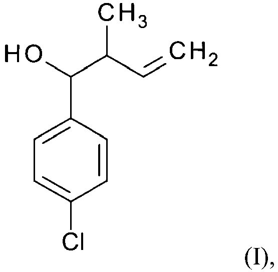 with bromochloromethane in the presence of (i) elemental zinc, (ii) catalytically active amounts of elemental copper and/or copper(I) compounds and/or copper(II) compounds, and (iii) at least one solvent, **characterized in that** the reaction of the alkene takes place without addition of organosilicon compounds, preferably without addition of haloalkylsilanes, and the elemental zinc has a lead content of not more than 0.005% by weight, preferably of not more than 0.002% by weight.

2. Process according to Claim 1, wherein the elemental zinc has a particle size distribution D90ₘₐₛₛ of not more than 0.5 mm.

3. Process according to either of Claims 1 or 2, wherein the elemental zinc has a total content of other metals of not more than 1% by weight.

4. Process according to any of Claims 1 to 3, wherein the elemental copper has a total content of other metals of not more than 1% by weight.

5. Process according to any of Claims 1 to 4, wherein the copper(I) compounds are selected from copper(I) chloride, copper(II) chloride, copper(II) phosphate, copper(II) carbonate or mixtures thereof.

6. Process according to any of Claims 1 to 5, wherein the haloalkylsilane is a chlorotrialkylsilane.

7. Process according to any of Claims 1 to 6, wherein the chlorotrialkylsilane is selected from chlorotrimethylsilane, chlorotriethylsilane, chlorotributylsilane, chlorotriisobutylsilane, chlorotrihexylsilane, and mixtures thereof.

8. Process according to any of Claims 1 to 7, wherein the at least one solvent is an ether, preferably selected from diethyl ether, 1,2-dimethoxyethane, methyl tert-butyl ether, tetrahydrofuran, cyclopentyl methyl ether, and mixtures thereof, and/or an aromatic hydrocarbon, preferably toluene.

9. Process according to any of Claims 1 to 8, wherein the bromochloromethane has a purity of at least 98% by weight and/or a water content of less than 0.02% by weight.

10. Process according to any of Claims 1 to 9, wherein from 1.5 to 4 mol, preferably from 2 to 3 mol, of metallic zinc based on 1.0 mol of the alkene, preferably based on 1.0 mol of the compound of formula (I), is used.

11. Process according to any of Claims 1 to 10, wherein the catalytically active amount of elemental copper and/or copper(I) compounds and/or copper(II) compounds is used in a total amount of from 0.001 to 0.1 mol, preferably from 0.001 to 0.01 mol, based on 1.0 mol of the alkene, preferably based on 1.0 mol of the compound of formula (I).

12. Process according to any of Claims 1 to 11, wherein from 1 to 3 mol, preferably from 1.7 to 2.2 mol, of bromochloromethane based on 1.0 mol of the alkene, preferably based on 1.0 mol of the compound of formula (I), is used.

13. Process according to any of Claims 1 to 12, wherein the reaction temperature is from 55 to 85°C, preferably from 60 to 80°C, and more preferably during and after the addition of bromochloromethane from 65 to 75°C.

14. Process according to any of Claims 1 to 13, wherein the alkene, preferably compound of formula (I), bromochloromethane, elemental zinc, catalytically active amounts of elemental copper and/or copper(I) compounds and/or copper(II) compounds, and at least one solvent, are mixed together to prepare the cyclopropyl compound, preferably the compound of formula (II), giving rise to a crude mixture, and the crude mixture is hydrolysed at the end of the preparation.

15. Process according to any of Claims 1 to 14, wherein the preparation of the cyclopropyl compound, preferably of the compound of formula (II), takes place in the presence of 0.1% to 5% by weight, based on the total weight of the crude mixture, of a crude mixture from a previous preparation of the cyclopropyl compound, preferably of the compound of formula (II), based on the amount of alkene used, preferably based on the amount of compound of formula (I) used.

## Revendications

1. Procédé pour la préparation de composés cyclopropyle de formule (II), 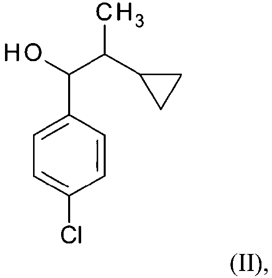 comprenant la mise en réaction d'un alcène de formule (I), 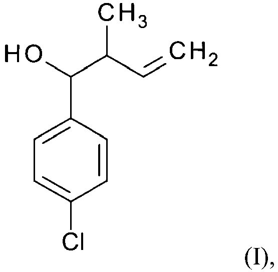 avec du bromochlorométhane en présence (i) de zinc élémentaire, (ii) de quantités catalytiquement actives de cuivre élémentaire et/ou de composés de cuivre(I) et/ou de composés de cuivre(II), et (iii) d'au moins un solvant, **caractérisé en ce que** la réaction de l'alcène s'effectue sans addition de composés organosiliciés, de préférence sans addition d'halogénoalkylsilanes, et le zinc élémentaire a une teneur en plomb d'au maximum 0,005 % en poids de préférence une teneur en plomb d'au maximum 0,002 % en poids .

2. Procédé selon la revendication 1, dans lequel le zinc élémentaire présente une distribution de tailles de particules D90ₘₐₛₛₑ non supérieure à 0,5 mm.

3. Procédé selon l'une quelconque des revendications 1 et 2, dans lequel le zinc élémentaire a une teneur totale en autres métaux d'au maximum 1 % en poids.

4. Procédé selon l'une quelconque des revendications 1 à 3, dans lequel le cuivre élémentaire a une teneur totale en autres métaux d'au maximum 1 % en poids.

5. Procédé selon l'une quelconque des revendications 1 à 4, dans lequel les composés de cuivre(I) sont choisis parmi le chlorure de cuivre(I), le chlorure de cuivre(II), le phosphate de cuivre(II), le carbonate de cuivre(II) ou des mélanges de ceux-ci.

6. Procédé selon l'une quelconque des revendications 1 à 5, dans lequel l'halogénoalkylsilane est un chlorotrialkylsilane.

7. Procédé selon l'une quelconque des revendications 1 à 6, dans lequel le chlorotrialkylsilane est choisi parmi le chlorotriméthylsilane, le chlorotriéthylsilane, le chlorotributylsilane, le chlorotri-isobutylsilane, le chlorotrihexylsilane et des mélanges de ceux-ci.

8. Procédé selon l'une quelconque des revendications 1 à 7, dans lequel ledit au moins un solvant est un éther, de préférence choisi parmi l'éther diéthylique, le 1,2-diméthoxyéthane, l'éther méthyl-tert-butylique, le tétrahydrofurane, l'éther cyclopentylméthylique et des mélanges de ceux-ci, et/ou un hydrocarbure aromatique, de préférence le toluène.

9. Procédé selon l'une quelconque des revendications 1 à 8, dans lequel le bromochlorométhane présente une pureté d'au moins 98 % en poids et/ou une teneur en eau inférieure à 0,02 % en poids.

10. Procédé selon l'une quelconque des revendications 1 à 9, dans lequel sont utilisées de 1,5 à 4 moles, de préférence de 2 à 3 moles, de zinc métallique par rapport à 1,0 mole de l'alcène, de préférence par rapport à 1,0 mole du composé de formule (I).

11. Procédé selon l'une quelconque des revendications 1 à 10, dans lequel la quantité catalytiquement efficace de cuivre élémentaire et/ou de composés de cuivre(I) et/ou de composés de cuivre(II), est utilisée en une quantité totale de 0,001 à 0,1 mole, de préférence de 0,001 à 0,01 mole, par rapport à 1,0 mole de l'alcène, de préférence par rapport à 1,0 mole du composé de formule (I).

12. Procédé selon l'une quelconque des revendications 1 à 11, dans lequel sont utilisées de 1 à 3 moles, de préférence de 1,7 à 2,2 moles, de bromochlorométhane par rapport à 1,0 mole de l'alcène, de préférence par rapport à 1,0 mole du composé de formule (I).

13. Procédé selon l'une quelconque des revendications 1 à 12, dans lequel la température de réaction est de 55 à 85 °C, de préférence de 60 à 80 °C, de façon particulièrement préférée de 65 à 75°C pendant et après l'addition de bromochlorométhane.

14. Procédé selon l'une quelconque des revendications 1 à 13, dans lequel l'alcène, de préférence un composé de formule (I), le bromochlorométhane, le zinc élémentaire, des quantités catalytiquement actives de cuivre élémentaire et/ou de composés de cuivre(I) et/ou de composés de cuivre (II), et au moins un solvant, sont mélangés pour la préparation du composé cyclopropyle, de préférence du composé de formule (II), de sorte qu'il en résulte un mélange brut, et une fois la préparation terminée le mélange brut est hydrolysé.

15. Procédé selon l'une quelconque des revendications 1 à 14, dans lequel la préparation du composé cyclopropyle, de préférence du composé de formule (II), s'effectue en présence de 0,1 à 5 % en poids, par rapport au poids total du mélange brut, d'un mélange brut provenant d'une préparation précédente du composé cyclopropyle, de préférence du composé de formule (II), par rapport à la quantité utilisée de l'alcène , de préférence par rapport à la quantité utilisée du composé de formule (I).
